# EUROPEAN PATENT SPECIFICATION

(11) **EP 0 635 208 B1**
(45) Date of publication and mention of the grant of the patent: **02.10.2002**
(21) Application number: 94305277.9
(22) Date of filing: 19.07.1994
(51) Int. Cl.: A01N 35/04, A01N 31/14, A01N 37/40, A61K 31/11, A61K 31/19, A61K 31/085, A61K 31/235

(54) **Louse repellent compositions**
Lausabweisende Zusammensetzungen
Compositions répulsives contre les poux

(30) Priority: 19.07.1993 GB 9314929
(43) Date of publication of application: 25.01.1995
(73) Proprietor: UNICLIFFE LIMITED, Stonar, Kent CT13 9NJ (GB)
(72) Inventor: Irwin, Richard Neil, Nr. Alton, Hampshire GU 34 5ES (GB)
(74) Representative: Brookes Batchellor

(56) References cited:
- FR-A- 2 417 257
- GB-A- 488 519
- US-A- 1 963 955
- US-A- 2 400 006
- R.WEGLER: "Chemie der Pflanzenschutz- und Schädlingsbekämpfungsmittel", 1970, SPRINGER-VERLAG, BERLIN

## Description

The invention relates to louse repellent compositions.

US 2 400 006 describes the use of ortho and para alkoxyphenylalkyl alcohols of specified structure as repellent for Trombiculidae, Aedes aegypti, Aedes taeniorhynchus and Anopheles quadrimaculatus.

We have surprisingly found that a range of methoxy aromatic compounds have louse repellent and pediculicidal properties.

According to the invention there is provided the use of a compound of general formula I in the manufacture of a medicament having pediculicidal or louse repellent properties. 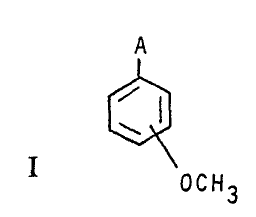 A is -CH₂OR, -CHO, -CC₂R¹
wherein
R is H, C₁ to C₅ alkyl or 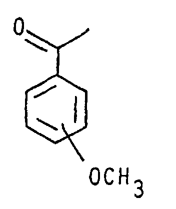 and
R¹ is H or C₁ to C₃ alkyl

The para isomer is often somewhat preferred on cost grounds but ortho and meta isomers are both usable.

Some of the compounds such as p-anisaldehyde have agreeable odours and may be preferred.

Preferred compounds of the invention include 4-methoxybenzaldehyde (II), 4-methoxybenzoic acid (III), 4-methoxybenzyl alcohol (IV) and p-anisyl p-anisicate (V)

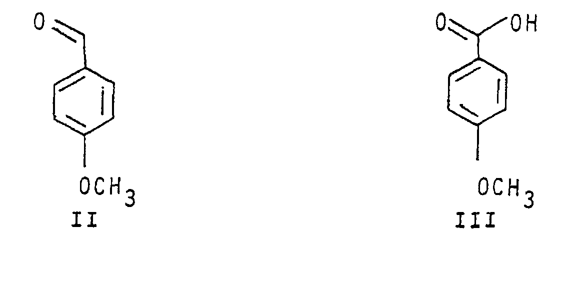

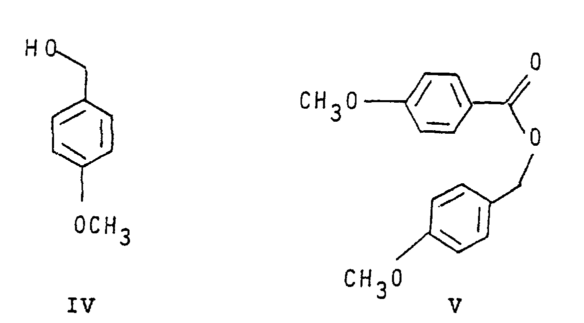

At least some of the compounds are readily available commercially. Those skilled in the art will readily be able to obtain others.

The active ingredient is conveniently applied directly to the hair. Those skilled will readily be able to devise vehicles for doing this. Convenient forms include lotions gels and mousses which may be prepared in a manner familiar to those skilled in the art.

Preferably the compounds are presented in alcoholic solution. The alcohol may be ethanol, propanol especially isopropanol, other alcohols or mixtures thereof. Preferably aqueous alcohol is used to reduce the possibility of skin irritation.

The efficiency of the composition may be enhanced by ensuring that it has a tendency to coat the hair. This can be achieved by incorporating spreading film formers such as silicones, soluble lanolin or soluble polymers such as PVP optionally copolymerised with vinyl acetate.

Many of the compounds of the invention are solid at room temperature and may have a tendency to crystallise from alcohol solution at low temperature eg about 0°C. The crystals may block the dispenser. This problem can be substantially reduced by incorporating an emulsifier.

Preferably two emulsifiers are employed. A, the or each emulsifier may be an nonionic emulsifier such as alkoxylated fatty alcohol or fatty acid. The emulsifier keeps the active ingredient in solution and deters crystallisation. Where two emulsifiers are used the solubilisation appears to be increased synergistically. Where emulsifier is present the active ingredient tends to form a film rather than to crystallise rapidly in use.

High alcohol concentrations are preferably avoided to reduce the possibility of irritation. Furthermore the comparatively high water content makes the composition pleasant to use. Preferred concentrations of active are from about 0.5 to 1 wt% more preferably 1 to 3 wt%. The compositions may also preferably contain phenoxyethanol as an antimicrobial preservative.

Examples of the invention will now be described by way of illustration.

### Example 1

An aqueous alcoholic mousse was prepared by heating to 60°C all the following components other than the propellant. The warm mixture was transferred to aerosol containers and charged with propellant in the usual way.

| | wt% |
|---|---|
| p-Anisaldehyde | 2-10 |
| Ethanol | 50 |
| Water | qs |
| Propylene glycol | 5 |
| Polawax A31 | 5 |

### Propellant

Polawax A31 is an ethoxylated cetostearyl alcohol oil in water emulsifying wax. It is designated Ceteareth-20 by the CTFA.

### Example 2

An aqueous alcoholic lotion was prepared by mixing the following components:

| | wt% |
|---|---|
| p-Anisaldehyde | 2-10 |
| Eumulgin RO40 | 1-10 |
| Eumulgin L | 5-30 |
| Water | qs |

Eumulgin RO40 is an ethoxylated castor oil and Eumulgin L is a 2-hydroxy fatty acid alkoxylate. Both are available from Henkel AG.

### Example 3

An aqueous alcoholic lotion was prepared by mixing the following ingredients:

| | wt% |
|---|---|
| p-Anisaldehyde | 2-10 |
| Dow Corning 190 | 0.5-2.0 |
| Ethanol | 15-40 |
| Isopropanol | 5-30% |
| Water | qs |

Dow Corning 190 is modified silicone fluid.

### Example 4

An aqueous alcoholic lotion was prepared in a similar manner to the lotion of Example 3. However the Dow Corning 190 was replaced by 1-3 wt% of the soluble lanolin Lanexol AWS.

### Example 5

An aqueous alcoholic lotion was prepared in the same way as the lotion of Example 3. However the Dow Corning 190 was replaced by 1-3 wt% of a soluble polymer of the Kollidon series which are polyvinylpyrollidones which are copolymerised in some cases with vinyl acetate.

## Claims

1. The use of a compound of general formula I in the manufacture of a medicament having pediculicidal or louse repellent properties 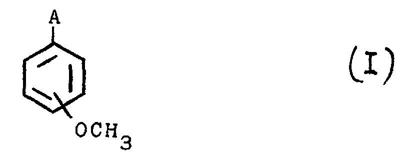 A is =CH₂OR, -CHO, -CO₂R¹
wherein
R is H, C₁ to C₅ alkyl or 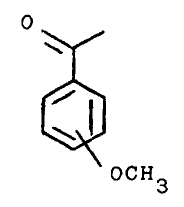 and
R¹ is H or C₁ to C₃ alkyl

2. The use as claimed in claim 1 wherein the compound of general formula I is 4-methoxybenzaldehyde, 4-methoxybenzoic acid, 4-methoxybenzyl alcohol or p-anisyl p-anisicate.

3. The use as claimed in claim 2 wherein the compound of general formula I is 4-methoxybenzaldehyde.

4. A pediculicidal or louse repellent composition comprising:
i) at least 0.5 wt. % of a compound of formula I
wherein R is C₁ to C₅ alkyl
ii) aqueous alcohol, and
iii) an emulsifier, or
iv) a spreading film former.

5. A composition as claimed in claim 4 wherein the emulsifier comprises ethoxylated castor oil fatty acids.

6. A composition as claimed in claim 4 or 5 wherein component iv) is a silicone, lanolin or polyvinylpyrrolidone.

7. A composition as claimed in any one of claims 4 to 6 wherein the compound of formula I is 4-methoxybenzaldehyde.

## Patentansprüche

1. Verwendung einer Verbindung der allgemeinen Formel I bei der Herstellung eines Medikaments mit pedikuliziden oder lausabweisenden Eigenschaften
A ist -CH₂OR, -CHO, CO₂R¹ 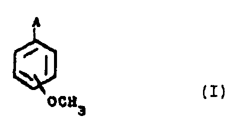 worin
R H, C₁-C₅-Alkyl oder 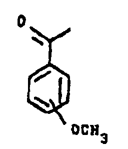 ist
und
R¹ H oder C₁-C₃-Alkyl ist.

2. Verwendung nach Anspruch 1, wobei die Verbindung der allgemeinen Formel I 4-Methoxybenzaldehyd, 4-Methoxybenzoesäure, 4-Methoxybenzylalkohol oder p-Anisyl-p-methoxybenzoat ist.

3. Verwendung nach Anspruch 2, wobei die Verbindung der allgemeinen Formel 1 4-Methoxybenzaldehyd ist.

4. Pedikulizide oder lausabweisende Zusammensetzung, umfassend:
i) zu wenigstens 0,5 Gew.-% eine Verbindung der Formel I, worin R C₁-C₅-Alkyl ist,
ii) wässerigen Alkohol und
iii) einen Emulgator oder
iv) einen verteilenden Filmbildner.

5. Zusammensetzung nach Anspruch 4, wobei der Emulgator ethoxylierte Ricinusöl-Fettsäuren umfaßt.

6. Zusammensetzung nach Anspruch 4 oder 5, wobei Bestandteil iv) ein Silikon, Lanolin oder Polyvinylpyrrolidon ist.

7. Zusammensetzung nach einem der Ansprüche 4 bis 6, wobei die Verbindung der Formel I 4-Methoxybenzaldehyd ist.

## Revendications

1. Utilisation d'un composé de formule générale I dans la production d'un médicament ayant des propriétés répulsives anti-pédiculose et anti-poux 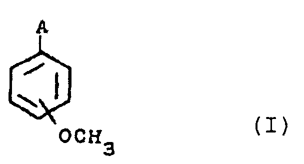 A est =CH₂OR, -CHO, -CO₂R¹
dans laquelle R est un H
ou un alkyle en C₁ à C₅ ou 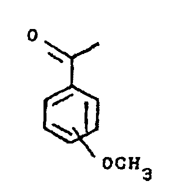 et R¹ est un H ou un alkyle en C₁ à C₃.

2. Utilisation selon la revendication 1, dans laquelle le composé de formule générale I est le 4-methoxybenzaldéhide, l'acide 4-methoxybenzoïque, l'alcool méthoxybenzilique ou le p-anisyl p-anisicate.

3. Utilisation selon la revendication 2, dans laquelle le composé de formule générale I est du 4-methoxybenzaldéhide.

4. Composition répulsive anti-pédiculose et anti-poux comprenant :
i) au moins 0,5 % en poids d'un composé de formule I dans laquelle R est un alkyle en C₁ à C₅
ii) un alcool aqueux et
iii) un agent émulsifiant ou
iv) un agent d'enduction filmogène.

5. Composition selon la revendication 4, dans laquelle l'agent émulsifiant comprend des acides gras d'huile de castor éthoxylée.

6. Composition selon la revendication 4 ou 5, dans laquelle iv) est de la silicone, de la lanoline, ou du polyvinylpyrrolidone.

7. Composition selon l'une quelconque des revendications 4 à 6, dans laquelle le composé de formule I est du 4-methoxybenzaldéhide.
